(19) Europäisches Patentamt
European Patent Office
Office européen des brevets

(11) Veröffentlichungsnummer: **0 116 942**
**B1**

# (12) EUROPÄISCHE PATENTSCHRIFT

(45) Veröffentlichungstag der Patentschrift: **16.07.86**

(51) Int. Cl.⁴: **C 07 D 263/62**, C 07 D 277/66, C 07 D 235/20

(21) Anmeldenummer: **84101506.8**

(22) Anmeldetag: **14.02.84**

## (54) Verfahren zur Herstellung von 4,4'-Bis-benz-ox(-thi, -imid)-azol-2-yl-stilbenen.

(30) Priorität: **18.02.83 DE 3305578**

(43) Veröffentlichungstag der Anmeldung: **29.08.84 Patentblatt 84/35**

(45) Bekanntmachung des Hinweises auf die Patenterteilung: **16.07.86 Patentblatt 86/29**

(84) Benannte Vertragsstaaten: **BE CH DE FR GB IT LI NL SE**

(56) Entgegenhaltungen:
**DE - A - 2 948 183**
**GB - A - 1 026 368**
**US - A - 4 156 778**
**US - A - 4 282 355**

(73) Patentinhaber: **HOECHST AKTIENGESELLSCHAFT, Postfach 80 03 20, D-6230 Frankfurt am Main 80 (DE)**
(84) Benannte Vertragsstaaten: **CH DE FR GB IT LI NL**

(73) Patentinhaber: **Heiss, Lorenz, Dr., Stormstrasse 39, D-6238 Hofheim am Taunus (DE)**
(84) Benannte Vertragsstaaten: **BE SE**

(72) Erfinder: **Heiss, Lorenz, Dr., Stormstrasse 39, D-6238 Hofheim am Taunus (DE)**

(74) Vertreter: **Meier, Karl, Dr. et al, Hoechst AG Zentrale Patentabteilung Postfach 80 03 20, D-6230 Frankfurt am Main 80 (DE)**

**EP 0 116 942 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Aus Khim. Geterotrikl. Soedin 1981, S. 4 4 ist bekannt, dass man bei der Reaktion von Brommethylbenzoxazol-2-yl-benzol mit der vierfachen Menge an Kaliumhydroxid in Dimethylformamid in einer Ausbeute von 51% den 4,4'-Bis-(benzoxazol-2-yl)-dibenzylether erhält. Es wurde nun überraschend gefunden, dass diese Reaktion gänzlich anders verläuft und zu den als optische Aufheller sehr bedeutsamen Bis-benzoxazol-stilbenen führt, wenn man nicht von der Brommethyl- sondern von der Chlormethylverbindung ausgeht.

Die Herstellung von Di-benz-ox(-thi)-azol-2-yl-stilbenen durch Kondensation von 4,4'-Stilben-dicarbonsäuren mit den entsprechenden o-Hydroxyarylaminen oder o-Mercapto-arylaminen ist im GB-A-1 026 368 beschrieben.

Gegenstand der Erfindung ist somit ein Verfahren zur Herstellung von 4,4-Bis-benz-ox(-thi, -imid)-azol-2-yl-stilbenen der Formel

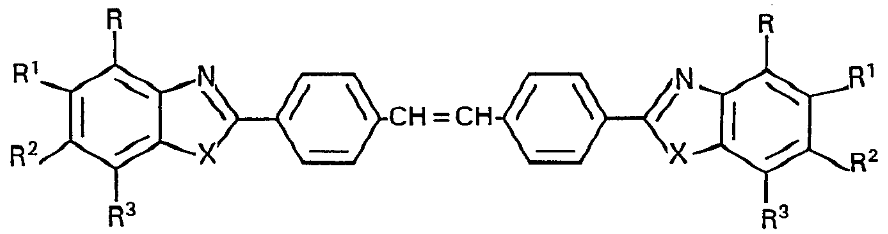

worin R, $R^1$, $R^2$ und $R^3$ gleich oder verschieden sind und Wasserstoff, $C_1$-$C_4$-Alkyl, $C_1$-$C_4$-Alkoxy, $C_1$-$C_4$-Alkoxycarbonyl, Chlor, Brom, Nitro oder Sulfo und X Sauerstoff, Schwefel oder NH bedeuten, das darin besteht, dass man (4-methyl)-iminobenzoesäurealkylester der Formel

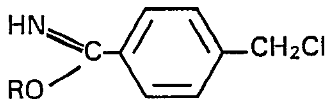

oder deren HCl-Salze, wobei R $C_1$-$C_4$-Alkyl oder $C_1$-$C_4$-Alkoxyethyl bedeutet, mit o-Aminophenol, o-Aminothiophenol oder o-Phenylendiamin umsetzt und die dabei erhaltenen 2-Benz-ox(-thi, -imid)-azolyl-4-methylbenzole mit Kalium- oder Natriumhydroxid in einem polaren aprotischen Lösungsmittel umsetzt.

Die als Ausgangsverbindungen dienenden (4-Chlormethyl)-iminobenzoesäurealkylester werden hergestellt, indem man p-Cyanbenzylchlorid in einem Überschuss des Alkohols der Formel R-OH dispergiert und Chlorwasserstoff einleitet. Man erhält so die HCl-Salze der Imino-ester der oben angegebenen Formel.

Diese Salze werden dann mit einem o-Aminophenol, o-Aminothiophenol oder o-Phenylendiamin umgesetzt und zwar durch Erhitzen in einem inerten organischen Lösemittel, vorzugsweise in einem niederen Alkohol, bei Temperaturen von Raumtemperatur bis zur Siedetemperatur des Lösemittels. Nach Beendigung der Reaktion gibt man Wasser zu, saugt das Reaktionsprodukt ab und wäscht mit Wasser nach. Man erhält so das 2-Benzoxazolyl-4-chlormethylbenzol bzw. die entprechenden Benzthiazolyl- oder Benzimidazolyl-Verbindungen.

Zur Herstellung der Stilben-Verbindungen werden diese Chlormethylbenzol-Verbindungen in einem wasserfreien aprotischen polaren Lösungsmittel, vorzugsweise in Dimethylformamid gelöst und durch Zugabe von pulverförmigem Natrium oder Kaliumhydroxid dimerisiert. Die Menge an Alkalihydroxid soll ungefähr 2 bis 4 Mol betragen auf ein Mol der Chlormethylverbindung. Damit die Reaktion glatt abläuft, wird das Alkalihydroxid als Pulver eingesetzt. Die Reaktion wird bei Raumtemperatur durchgeführt und zwar so lange, bis alles organisch gebundene Chlor reagiert hat. Um das durch die Neutralisation entstandene Reaktionswasser zu binden, kann es von Vorteil sein, Verbindungen wie etwa $Na_2O$, CaO, MgO oder NaH zuzugeben. Dadurch wird eine Steigerung der Ausbeute erreicht.

Im allgemeinen beträgt die Reaktionszeit 6 bis 12 Stunden. Nach Beendigung der Reaktion säuert man mit einer Säure an, vorzugsweise mit einer wasserfreien organischen Säure wie etwa Eisessig. Zur Aufarbeitung erhitzt man dann das Reaktionsgemisch zum Sieden, um Nebenprodukte in Lösung zu bringen, und saugt heiss ab. Dann wird mit Lösemitteln und mit Wasser gewaschen, bis im Waschwasser keine Chlorid-ionen mehr nachweisbar sind. Nach dem Trocknen erhält man die analysenreine Stilbenverbindung in einer Ausbeute von ca. 90%.

Diese Stilbenverbindungen der eingangs aufgeführten Formel werden als optische Aufheller eingesetzt. Durch das hier beschriebene Verfahren lassen sich diese Verbindungen kostengünstig in hohen Ausbeuten herstellen.

*Beispiel*

75,8 g (0,5 Mol) p-Cyanbenzylchlorid werden in 32 g (1 Mol) Methanol dispergiert und bei 5° - 15°C ca. 35,5 g (1 Mol) Chlorwasserstoff eingeleitet. Man rührt ca. 20 Stunden nach bis im Infrarot-spektrum die Nitrilbande bei 2220 $cm^{-1}$ verschwunden ist. Nach Entfernung des Methanols und Chlorwasserstoffs erhält man 110 g (0,5 M) 4-Chlormethyl-benzimidomethylether-hydrochlorid.

Ausbeute: quantitativ

| | | | | |
|---|---|---|---|---|
| gefunden: | C 49,0 | H 5,0 | Cl 32,0 | $Cl^-$ 16,1 |
| berechnet: | C 49,2 | H 5,0 | Cl 32,2 | $Cl^-$ 16,1 |

110 g (0,5 m) 4-Chlormethyl-iminobenzoesäure-methylesterhydrochlorid werden in 500 g Methanol dispergiert und 54,5 g (0,5 m)-o-Amino-phenol und

120 g Eisessig zugesetzt. Man kocht unter Rückfluss bei 65°C eine Stunde und wäscht mit 30 g Methanol, dann mit Wasser nach, bis das Waschwasser von Chlorionen frei ist. Nach dem Trocknen erhält man 117 g (0,48 mol) 4-Chlormethyl-benzoxazol-2-yl-benzol

Ausbeute: 96% d.Th.

Schmelzpunkt: 149°C

| | | | |
|---|---|---|---|
| gefunden: | C 69,0 | H 4,1 | Cl 14,6 |
| berechnet: | C 69,1 | H 4,1 | Cl 14,6 |

In 150 g Dimethylformamid werden unter Stickstoffatmosphäre 16 g (0,4 Mol) Natriumhydroxydpulver unter Rühren dispergiert, bei 20°C 24,4 g (0,1 Mol) 4-Chlormethylbenzoxazol-2-yl-benzol eingetragen und ca. 6 - 12 h gerührt bis alles organisch gebundene Chlor reagiert hat.

Man säuert mit Essigsäure an, erhitzt auf Rückfluss, saugt unter 100°C ab, wäscht mit Dimethylformamid, dann mit Wasser bis Waschwasser von Chlorionen frei ist und trocknet. Man erhält in grüngelb fluoreszierenden Kristallen 4,4'-Bisbenzoxazol-2-yl-Stilben in einer Ausbeute von 18,6 g (89,8% d.Th.).

Schmelzpunkt: 355 - 360°C.

| | | | |
|---|---|---|---|
| gefunden: | C 80,8 | H 4,3 | N 6,8 |
| berechnet: | C 81,1 | H 4,3 | N 6,8 |

Unter den gleichen Reaktionsbedingungen wurden die in der folgenden Tabelle aufgeführten Endprodukte erhalten.

Tabelle 1

| Beispiel | Ausgangsprodukte | Endprodukte | Schmelzpunkt | C, H, Cl-Analyse | Ausbeute |
|---|---|---|---|---|---|
| 2) | 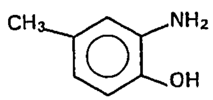 | 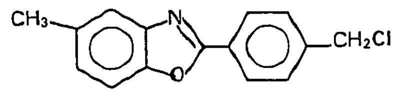 | 144°C | gef. 69,7%; 4,8%; 13,5%<br>ber. 69,9%; 4,7%; 13,8% | 91% |
| 3) | 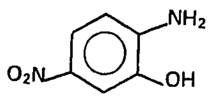 | 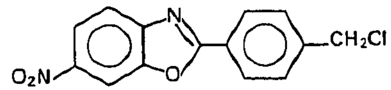 | 138°C | gef. 58,1%; 3,3%; 11,9%<br>ber. 58,2%; 3,1%; 12,2% | 88% |
| 4) | 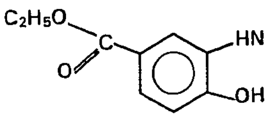 | 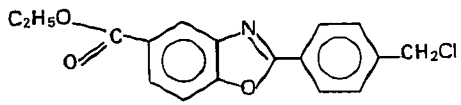 | 137°C | gef. 64,6%; 4,4%; 11,0%<br>ber. 64,8%; 4,4%; 11,3% | 82% |
| 5) | 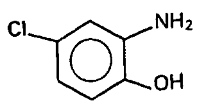 | 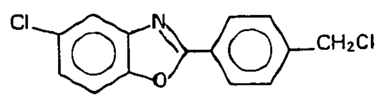 | 167°C | gef. 60,0%; 3,3%; 24,9%<br>ber. 60,3%; 3,2%; 25,5% | 86% |
| 6) | 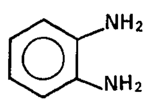 | 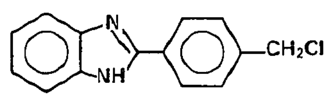 | subl.<br>200°C | gef. 68,9%; 4,8%; 14,2%<br>ber. 69,2%; 4,6%; 14,6% | 90% |
| 7) | 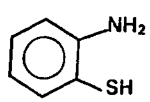 | 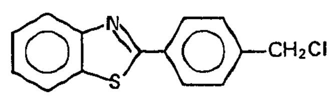 | 114°C | gef. 64,3%; 3,8%; 13,5%<br>ber. 64,9%; 3,9%; 13,7% | 56% |
| 8) | 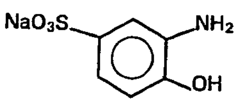 | 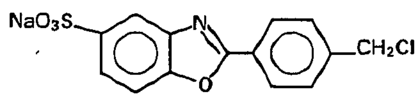 | — | gef. 48,3%; 2,7%; 10,2%<br>ber. 48,7%; 2,6%; 10,2% | 51% |

Tabelle 2

| Beispiel | Ausgangsprodukte | Endprodukte | Schmelzpunkt | C, H, N-Analyse | Ausbeute |
|---|---|---|---|---|---|
| 2) | 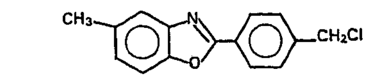 | 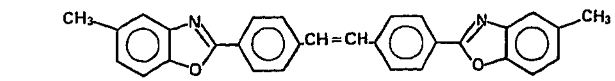 | 280-285°C | gef. 80,8%; 5,1%; 6,2%<br>ber. 81,2%; 5,2%; 6,3%<br>81,5%; 5,0% | 94% |
| 3) | 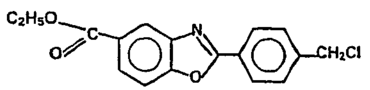 | 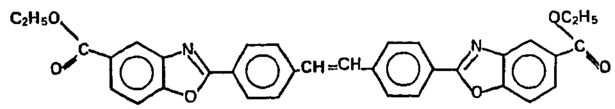 | 272-278°C | gef. 72,8%; 4,8%; 5,1%<br>ber. 73,1%; 4,7%; 5,0% | 88% |
| 4) | 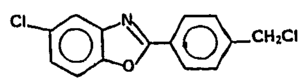 | 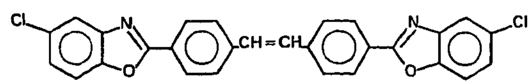 | 306-310°C | gef. 69,1%; 3,3%; 5,7%<br>ber. 69,6%; 3,3%; 5,8% | 91% |
| 5) | 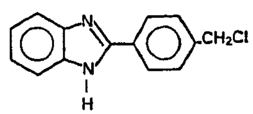 | 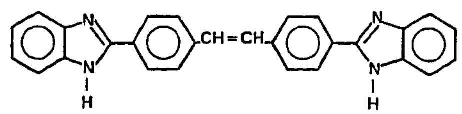 | > 340°C<br>Zers. | gef. 81,2%; 4,7%; 13,5%<br>ber. 81,6%; 4,8%; 13,6% | 86% |
| 6) | 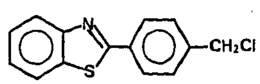 | 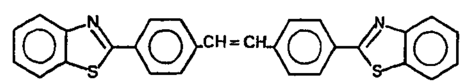 | 302-308°C | gef. 75,0%; 4,1%; 3,1%<br>ber. 75,3%; 4,0%; 3,1% | 72% |
| 7) | 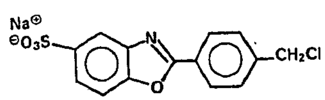 | 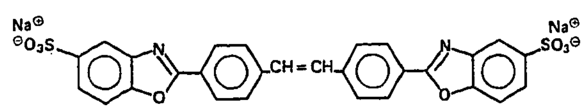 | > 360°C | gef. 53,9%; 2,5%; 4,4%<br>ber. 54,4%; 2,6%; 4,5% | 76% |
| 8) | 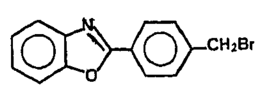 | 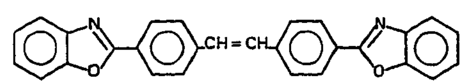 | 358-362°C | gef. 80,7%; 4,3%; 6,7%<br>ber. 81,1%; 4,3%; 6,8% | 90% |

**Patentanspruch**

Verfahren zur Herstellung von 4,4-Bis-benz-ox(-thi, -imid)-azol-2-yl-stilbenen der Formel

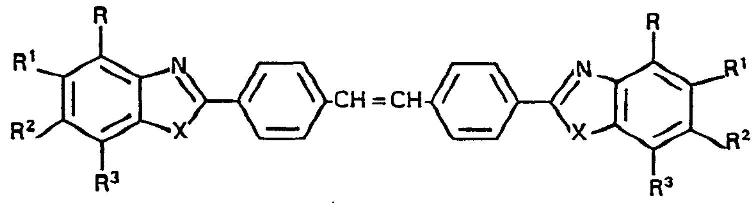

worin R, $R^1$, $R^2$ und $R^3$ gleich oder verschieden sind und Wasserstoff, $C_1$-$C_4$-Alkyl, $C_1$-$C_4$-Alkoxy, $C_1$-$C_4$--Alkoxycarbonyl, Chlor, Brom, Nitro oder Sulfo und X Sauerstoff, Schwefel oder NH bedeuten, dadurch gekennzeichnet, dass man (4-Chlormethyl)-imino-benzoesäurealkylester der Formel

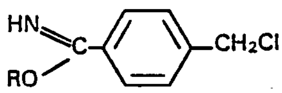

oder deren HCl-Salze, wobei R $C_1$-$C_4$-Alkyl oder $C_1$-$C_4$-Alkoxyethyl bedeutet, mit o-Aminophenol, o-Aminothiophenol oder o-Phenylendiamin umsetzt und die dabei erhaltenen 2-Benz-ox(-thi, -imid)-azolyl-4--methylbenzole mit Kalium- oder Natriumhydroxid in einem polaren aprotischen Lösungsmittel umsetzt.

**Claim**

A process for preparing 4,4'-bis-benz-ox(-thi,-imid)-
-azol-2-yl-stilbenes of the formula

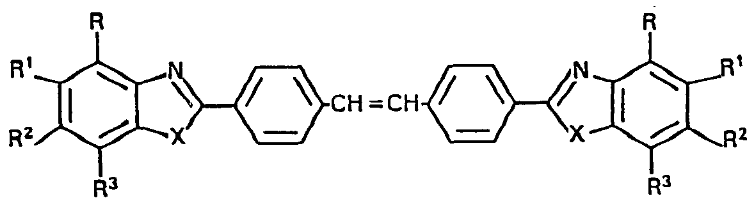

wherein R, $R^1$, $R^2$ and $R^3$ may be the same or different from each other and denote hydrogen, $C_1$-$C_4$-alkyl, $C_1$-$C_4$-alkoxy, $C_1$-$C_4$-alkoxycarbonyl, chlorine, bromine, nitro or sulfo and X denotes oxygen, sulfur or NH, which comprises reacting a (4-chlormethyl)--iminobenzoic acid alkyl ester of the formula

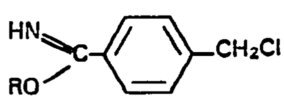

wherein R denotes $C_1$-$C_4$-alkyl or $C_1$-$C_4$-alkoxyethyl, or HCl salts thereof, with o-aminophenol, o-aminothiophenol or o-phenylene diamine and reacting the resulting 2-benz-ox(-thi, -imid)-azolyl-4-chloromethyl-benzenes with potassium hydroxide or sodium hydroxide in a polar aprotic solvent.

**Revendication**

Procédé de préparation de bis-(benzoxazolyl-2)--4,4' stilbènes, de bis-(benzothiazolyl-2)-4,4' stilbènes et de bis-(benzimidazolyl-2)-4,4' stilbènes qui répondent à la formule

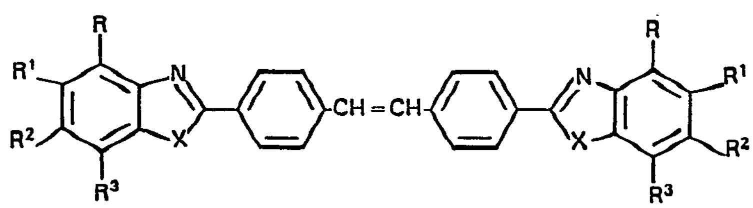

dans laquelle R, $R^1$, $R^2$ et $R^3$ sont identiques ou différents et représentent chacun l'hydrogène, un alkyle en $C_1$-$C_4$, un alcoxy en $C_1$-$C_4$, un alcoxycarbonyle dont la partie alcoxy contient de 1 à 4 atomes de carbone, le chlore, le brome, un nitro ou un sulfo, et X représente l'oxygène, le soufre ou NH, procédé caractérisé en ce qu'on fait réagir un chlorométhyl-4 benzène-carboximidate d'alkyle répondant à la formule

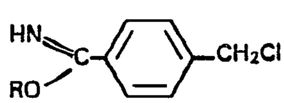

dans laquelle R représente un alkyle en $C_1$-$C_4$ ou un alcoxyéthyle dont la partie alcoxy contient de 1 à 4 atomes de carbone, ou son sel d'HCl, avec un o--amino-phénol, un o-amino-thiophénol ou une o--phénylène-diamine, et on fait réagir le (benzoxazolyl-2)-1 chlorométhyl-4 benzène ou le (benzthiazolyl-2)-1 chlorométhyl-4 benzène ou le (benzimidazolyl-2)-1 chlorométhyl-4 benzène ainsi obtenu avec l'hydroxyde de potassium ou l'hydroxyde de sodium dans un solvant aprotique polaire.